# EUROPEAN PATENT SPECIFICATION

(11) **EP 3 974 409 B1**
(45) Date of publication and mention of the grant of the patent: **23.07.2025**
(21) Application number: 20808857.5
(22) Date of filing: 12.05.2020
(51) Int. Cl.: C07C 29/128, C07C 29/80, C07C 27/02, C07C 68/065, C07C 31/20, C07C 69/96, B01J 29/80, B01J 23/02, B01J 23/04, B01J 21/04, B01J 29/08, B01J 37/02, B01J 37/08

(54) **METHOD AND APPARATUS FOR PRODUCING ETHYLENE GLYCOL USING ETHYLENE CARBONATE AND METHANOL**
VERFAHREN UND VORRICHTUNG ZUR HERSTELLUNG VON ETHYLENGLYKOL MIT ETHYLENCARBONAT UND METHANOL
PROCÉDÉ ET APPAREIL DE PRODUCTION D'ÉTHYLÈNE GLYCOL À L'AIDE DE CARBONATE D'ÉTHYLÈNE ET DE MÉTHANOL

(30) Priority: 22.05.2019 CN 201910427168
(43) Date of publication of application: 30.03.2022
(73) Proprietor: Shida Shinghwa Advanced Material Group Co., Ltd, Dongying, Shandong 257503 (CN)
(72) Inventor: JIA, Fenglei, Shandong 257503 (CN); GUO, Jianjun, Shandong 257503 (CN); LI, Xin, Shandong 257503 (CN); MA, Guo, Shandong 257503 (CN); LI, Guangke, Shandong 257503 (CN); GUO, Yunshan, Shandong 257503 (CN); CHANG, Guangkai, Shandong 257503 (CN)
(74) Representative: von Bülow & Tamada
(86) International application number: PCT/CN2020/089697
(87) International publication number: WO 2020/233441

(56) References cited:
- WO-A1-00/73256
- WO-A1-02/070452
- CN-A- 1 201 448
- CN-A- 109 651 153
- CN-A- 110 105 174
- JP-A- 2004 008 996
- JP-A- H06 343 871
- US-A- 4 661 609
- US-A- 4 691 041
- US-A- 4 691 041
- CROCELLA, VALENTINA ET AL.: "A multi-technique approach to disclose the reaction mechanism of dimethyl carbonate synthesis over amino-modified SBA-15 catalysts.", APPLIED CATALYSIS, B: ENVIRONMENTAL., vol. 211, 6 April 2017 (2017-04-06), pages 323 - 336, XP085011103, ISSN: 0926-3373
- XU, JIE ET AL.: "Mesostructured graphitic carbon nitride as a new base catalyst for the efficient synthesis of dimethyl carbonate by transesterification.", CATALYSIS SCIENCE & TECHNOLOGY., vol. 3, no. 12, 28 August 2013 (2013-08-28), pages 3192 - 3199, XP055755167, ISSN: 2044-4753

## Description

### FIELD

The present application relates to a method and apparatus for producing ethylene glycol using ethylene carbonate and methanol as raw materials, and belongs to the chemical industry.

### BACKGROUND

Ethylene glycol is an important glycol. It is mainly used to prepare polyester fiber, polyester resin, moisture absorbent, plasticizer, surfactant, synthetic fiber, cosmetics and explosives, and can be used as solvent for dyes, inks and the like, can be formulated into engine antifreeze, gas dehydrating agent, and can be used to prepare resin. It can also be used as wetting agent for cellophane, fiber, leather and adhesive. The preparation method thereof mainly comprises chloroethanol hydrolysis method, ethylene oxide hydration method, dimethyl oxalate hydrogenation reduction method (note: coal-to-ethylene glycol method), and transesterification method (using ethylene carbonate and lower alcohols as raw materials).

The transesterification method refers to the transesterification reaction of ethylene carbonate and methanol to produce ethylene glycol and dimethyl carbonate. Dimethyl carbonate is an important green solvent, and ethylene glycol is an important raw material for synthesizing fine chemical.

However, in the prior art, a homogeneous catalyst is used to produce ethylene glycol and dimethyl carbonate through the transesterification reaction of ethylene carbonate and methanol. The homogeneous catalyst, such as methanol solution of sodium methoxide or potassium methoxide, enters the reaction system in proportion along with raw materials. Part of the catalyst will react with the carbon dioxide produced by the decomposition of ethylene carbonate to be converted into sodium carbonate. Salts such as sodium methoxide and sodium carbonate are separated from the ethylene glycol in a column bottom in the EG rectification system, and a small amount of high-grade polyalcohol is entrained. These materials become solid wastes of chemical plants and need to be delivered to those companies with processing qualifications and processing capabilities to conduct harmless treatment.

Therefore, the use of homogeneous catalyst has the disadvantages of high consumption, high production cost, difficult post-treatment and generation of solid wastes. Under the premise that the environmental protection situation is becoming more and more severe, there is an urgent market demand for green environmental protection technology.

US4691041A describes a process for producing ethylene glycol by reacting ethylene carbonate with methanol in which a heterogenous catalyst is used.

### SUMMARY

According to one aspect of the present application, there is provided a method for producing ethylene glycol using ethylene carbonate and methanol as raw materials. The method involves a transesterification process using a solid catalyst and overcomes the disadvantages of high catalyst consumption, high production cost, difficult post-treatment and generation of solid wastes when a homogeneous catalyst is used.

A method for producing ethylene glycol using ethylene carbonate and methanol as raw materials comprising following steps:
a) performing pre-reaction of heated reactants to obtain a pre-treatment material, the reactants comprising ethylene carbonate and methanol;
b) subjecting the pre-treatment material to a catalytic rectification reaction to obtain a material stream I;
c) separating methanol from the material stream I to obtain a material stream II;
d) subjecting the material stream II to rectification to obtain a target product, the target product comprising ethylene glycol; wherein, the catalytic rectification reaction is performed in the presence of a catalyst I; the catalyst I comprising a solid catalyst.

Optionally, in step a), the pre-reaction is performed in the presence of catalyst II; wherein, the catalyst II comprises a solid catalyst.

The solid catalyst in the present application is heterogeneous catalyst.

Optionally, in step a), a weight hourly space velocity of the reactants, which is calculated as EC, ranges from 0.8 to 1.0 h⁻¹.

Optionally, in step a), a molar ratio of the ethylene carbonate to methanol ranges from 1:4 to 1:8.

Optionally, a heating temperature in step a) ranges from 65 to 100°C.

Optionally, in step b), a weight hourly space velocity of the pre-treatment material ranges from 0.15 to 0.21 h⁻¹.

Optionally, in step b), the catalytic rectification reaction is performed at a temperature ranging from 65 to 80°C.

Optionally, in step b), under the condition of introducing supplementary methanol, the pre-treatment material is subjected to a catalytic rectification reaction to obtain product I and azeotropic components, wherein the azeotropic components contain dimethyl carbonate and methanol.

Optionally, the azeotropic components are extracted from a column top of a reaction rectification column, and the reflux ratio of the azeotropic components ranges from 1:1 to 3:1.

Optionally, the shape of the solid catalyst is spherical or columnar.

Optionally, the solid catalyst is basic catalyst.

Optionally, the basic catalyst is a molecular sieve catalyst; and the molecular sieve catalyst comprises element-modified molecular sieve, wherein the element comprises at least one of an alkali metal element and an alkaline earth metal element, and the molecular sieve comprises at least one of X-type molecular sieve, and Y-type molecular sieve.

Specifically, in the molecular sieve catalyst, the content of the modification element ranges from 2 wt% to 3 wt%.

The molecular sieve catalyst (i.e., weak basic catalyst) is prepared by the following method:
mixing a mixture comprising a molecular sieve with a solution containing a modification element source, which is then subjected to drying to obtain a precursor I;
subjecting sequentially the precursor I to a first activation treatment and a second activation treatment to obtain the weak basic catalyst;
wherein, the first activation treatment is performed at a temperature ranging from 250 to 350°C for a time ranging from 6 to 10 h; and
the second activation treatment is performed at a temperature ranging from 450 to 550°C for a time ranging from 8 to 12h.

Optionally, the molecular sieve is X-type molecular sieve and Y-type molecular sieve; and the modification element is alkali metal element and alkaline earth metal element. In the present application, a molecular sieve consisting of X-type molecular sieve and Y-type molecular sieve is used, and the molecular sieve is modified with alkali metal element and alkaline earth metal element. As solid catalyst, it improves the selectivity of ethylene glycol to 97%.

Optionally, the weight ratio of the X-type molecular sieve to the Y-type molecular sieve ranges from 5:1 to 3:1.

Specifically, an alkaline earth metal ion comprises either calcium ion or magnesium ion.

Alkali metal ion comprises either sodium ion or potassium ion.

In a specific example, a mixture comprising X-type molecular sieve and Y-type molecular sieve is mixed with a solution comprising calcium ion and sodium ion, and dried to obtain the precursor I.

Optionally, the basic catalyst is a metal oxide catalyst, and the metal oxide catalyst comprises element-modified metal oxide, wherein the element comprises at least one of alkali metals.

The metal oxide catalyst (i.e., medium basic catalyst) is prepared by the following method:
mixing a material comprising a metal oxide with a solution comprising modification element source, which is subjected to drying to obtain a precursor II;
subjecting sequentially the precursor II to an activation treatment I and an activation treatment II to obtain the medium basic catalyst;
wherein, the activation treatment I is performed at a temperature ranging from 250 to 350°C for a time ranging from 6 to 10 h, and
the activation treatment II is performed at a temperature ranging from 500 to 600°C for a time ranging from 8 to 12 h.

Optionally, the metal oxide is γ-Al₂O₃. In the present application, alkali metal modified γ-Al₂O₃ is used as solid catalyst, which improves the selectivity of ethylene glycol to 97%.

Specifically, the content of the modification element in the metal oxide catalyst ranges from 5 wt% to 8 wt%.

In a specific example, the material comprising γ-Al₂O₃ is mixed with a solution comprising potassium ion and cesium ion, and dried to obtain the precursor II.

In the present application, in the process of preparing the catalyst, step-by-step activation treatment can achieve the effects of high conversion rate of ethylene carbonate (above 99%) and high selectivity of ethylene glycol (above 97%).

The molecular sieve catalyst is a weak basic catalyst, and the metal oxide catalyst is a medium basic catalyst.

In the present application, the element-modified metal oxide is used as the catalyst, which has the effect of simpler and more efficient preparation process, and higher conversion rate of ethylene carbonate and selectivity of ethylene glycol.

Optionally, in step c), the methanol is extracted from the column top of the methanol recovery column, and the reflux ratio of the methanol ranges from 0:1 to 1:1.

In the present application, the purpose of separating the methanol in the product I is to return the methanol to the raw material system for reuse.

Optionally, in step d), the product II is rectified under vacuum environment, with the purpose of lowering the temperature of the materials in the rectification system so that the temperature of the materials is lower than 150°C, avoiding the polymerization of the materials at high temperature and improving the yield of ethylene glycol ; wherein the vacuum environment ranges from 0 to 5KPa.

Optionally, in step d), after the ethylene glycol is rectified, the light components at column top (comprising ethylene glycol and methanol) are returned to the methanol recovery column to continue the separation so as to recover methanol and ethylene glycol separately, thereby reducing the consumption of methanol as raw material and increasing the yield of ethylene glycol.

Optionally, the content of the ethylene glycol in the target product is ≥99.5%.

The following specifically describes the preparation process of ethylene glycol comprising:
feeding ethylene carbonate (EC) and methanol (ME) as raw materials into a static mixer according to a preset molar ratio and mixing them uniformly, after preheating the obtained mixture to temperature in a range from 65 to 75°C in a preheater, feeding the preheated raw materials into a pre-reactor 3 to contact with a solid catalyst for performing reaction, then feeding the obtained pre-reaction products in a reaction rectification column to react with a solid catalyst in the column.

In the present application, the static mixer has the same function as a batching tank. Compared with the batching tank, it has the advantages of small size, saving equipment investment, not using mechanical stirring and saving power consumption and the like. Further, compared with the manner that feeding the raw materials separately into the preheater for preheating, the manner that mixing the raw materials in the static mixture uniformly before feeding the same in the preheater, achieves improved selectivity of ethylene glycol.

The filling rate of the solid catalyst in the reaction rectification column ranges from 10% to 50%. The dimethyl carbonate produced after reaction and the additional methanol supplemented from the column bottom form azeotropic components, which are extracted from the column top. The operating reflux ratio ranges from 1:1 to 3:1, most preferably is 1.3:1. The column bottom contains methanol (ME) and ethylene glycol (EG). The typical composition in the column bottom is ME in a range from 70% to 80% and EG in a range from 20% to 30%.

The methanol recovery temperature of the column top ranges from 60 to 65°C, the temperature of the column bottom ranges from 130 to 140°C, and the typical composition in the column reactor: ME 5.2%, EG 94.8%.

The packing in the reaction rectification column is CY700 gauze structured packing, with catalyst packing in the middle of the packing, and the number of theoretical plates ranges from 50 to 60.

The packing in the methanol recovery column is CY700 structured packing, and the number of theoretical plates ranges from 25 to 30.

The ethylene glycol rectification column is packed with stainless steel structured packing CY700, and the number of theoretical plates ranges from 50 to 60.

The temperature of the ethylene glycol rectification column ranges from 120 to 140°C, most preferably ranges from 122 to 130°C, the reflux ratio of the column top ranges from 1 to 3, most preferably ranges from 1.2 to 2.5, and typically is 1.4. The pressure at the column top is 1.2 KPa, and the pressure in the column reactor is 3.6 KPa.

The product extracted from the ethylene glycol rectification column 6 is cooled by a cooler and then extracted to a product intermediate tank. The EG content is ≥99.5%, typically is 99.78%. The extraction reflux ratio is 1.5:1.

Optionally, the method for producing ethylene glycol using ethylene carbonate and methanol as raw materials comprises following steps:
a) feeding the reactants heated by the preheater into the pre-reactor for pre-reaction to obtain the pre-treatment material, the reactants comprising ethylene carbonate and methanol;
b) feeding the pre-treatment material into the reaction rectification column for a catalytic rectification reaction to obtain the material stream I;
c) feeding the material stream I into a methanol recovery column to separate methanol in the material stream I to obtain the material stream II;
d) feeding the material stream II into the ethylene glycol rectification column for rectification to obtain a target product, the target product comprising ethylene glycol;

wherein, a catalytic rectification reaction is performed in the presence of a catalyst I;
the catalyst I comprising a solid catalyst.

Optionally, before heating the reactants, step a) further comprises mixing ethylene carbonate and methanol to obtain the reactants. In the present application, mixing ethylene carbonate and methanol before heating can improve the selectivity of ethylene glycol.

According to another aspect of the present application, there is provided an apparatus for producing ethylene glycol using ethylene carbonate and methanol. The apparatus comprises a raw material mixing unit, a preheating unit, a pre-reaction unit, a reaction rectification unit, a methanol recovery unit and an ethylene glycol rectification unit;
the raw material mixing unit, the preheating unit and the pre-reaction unit are connected in sequence;
an outlet of the pre-reaction unit is connected with an upper end of the reaction rectification unit;
a lower end of the reaction rectification unit is connected with an upper end of the methanol recovery unit;
a lower end of the methanol recovery unit is connected with the ethylene glycol rectification unit; and
the ethylene glycol rectification unit is provided with a target product outlet.

Specifically, the apparatus comprises a raw material mixer, a preheater, a pre-reactor, a reaction rectification column, a methanol recovery column, and an ethylene glycol rectification column;
the raw material mixer, the preheater and the pre-reactor are connected in sequence;
an outlet of the pre-reactor is connected with an upper end of the reaction rectification column;
a lower end of the reaction rectification column is connected with an upper end of the methanol recovery column;
a lower end of the methanol recovery column is connected with the ethylene glycol rectification column; and
a middle part of the ethylene glycol rectification column is provided with a target product outlet.

In the present application, the function of the preheater is to raise the temperature of the material to the temperature required for the reaction. Without the preheater, the reaction cannot occur; and the pre-reactor is provided to reduce the volume of the catalyst filled in the rectification column. The reduced amount of catalyst filled in the rectification column can reduce the height of the rectification column, thereby reducing equipment investment.

Optionally, the lower end of the reaction rectification column is also provided with a methanol-supplementing inlet.

Specifically, in the present application, the methanol-supplementing inlet is provided at the lower end of the reaction rectification column, which has the effect of making the methanol in the reaction rectification system excess and improving the conversion rate of ethylene carbonate.

Optionally, the upper end of the ethylene glycol rectification column is connected with the upper end of the methanol recovery column.

In the present application, by providing a communication pipeline between the upper end of the ethylene glycol rectification column and the upper end of the methanol recovery column, the light components in the ethylene glycol rectification column can be returned to the methanol recovery column, thereby increasing yield of ethylene glycol.

Optionally, the pre-reactor is filled with catalyst II; a filling volume percentage of the catalyst II in the pre-reactor ranges from 70% to 90%.

Optionally, the reaction rectification column is filled with catalyst I; a filling volume percentage of the catalyst I in the reaction rectification column ranges from 10% to 50%.

In the present application, the term "weight hourly space velocity of reactant" refers to a ratio of the mass flow rate of ethylene carbonate to the weight of catalyst II in the pre-reactor.

The term "weight hourly space velocity of pre-treatment material" refers to a ratio of the mass flow rate of ethylene carbonate to the mass of catalyst I in the reaction rectification column.

The beneficial effects that the present application can achieve comprise:
1) A method for producing ethylene glycol using ethylene carbonate and methanol as raw materials is provided in the present invention. The main purpose of the method is to produce ethylene glycol. The method provided in the present application achieves very high selectivity of ethylene glycol to greater than 97.0%.
2) In the present application, methanol and ethylene carbonate as raw materials are mixed and preheated, and then enter in the pre-reactor and the reaction rectification column. The pre-reactor and the reaction rectification column are filled with a solid catalyst to catalyze the transesterification reaction. Methanol and ethylene glycol in the column reactor enter in the methanol recovery column, and the crude ethylene glycol exiting from the methanol recovery column enters in the ethylene glycol rectification column to produce ethylene glycol as product. The method for producing ethylene glycol provided in the present invention generates very little solid wastes, about from 2 to 3 t/a, having the advantages of high production efficiency, few by-products, low cost and the like, and is suitable for large-scale industrial production.

### BRIEF DESCRIPTION OF DRAWINGS

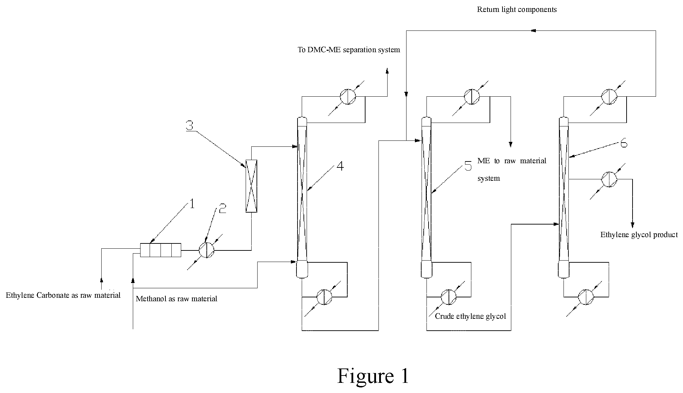

Figure 1 is a schematic structural diagram of an apparatus for producing ethylene glycol using ethylene carbonate and methanol as raw materials provided by possible embodiments in the present application.

List of parts and reference numerals:
1 static mixer; 2 preheater; 3 pre-reactor; 4 reaction rectification column; 5 methanol recovery column; 6 ethylene glycol rectification column;

In the Figure, DMC is the abbreviation for dimethyl carbonate, and ME is the abbreviation for methanol.

### DETAILED DESCRIPTION

The present application will be described in detail below with reference to the examples, but the present application is not limited to these examples. Unless otherwise specified, the raw materials in the examples of the present application are all commercially available.

The method and apparatus for producing ethylene glycol using ethylene carbonate and methanol as raw materials provided in the present application are as follows.
A. The apparatus comprises followings: a static mixer 1, a preheater 2, a pre-reactor 3, a reaction rectification column 4, a methanol recovery column 5, and an ethylene glycol rectification column 6, wherein the static mixer 1 is connected with the pre-reactor 3 via the preheater 2, the upper part of pre-reactor 3 is connected with the upper portion of reaction rectification column 4, the bottom of reaction rectification column 4 is connected with the upper part of methanol recovery column 5 via a process pipeline, and the lower part of the methanol recovery column 5 is connected with the middle part of the ethylene glycol rectification column 6,
B. The method comprises followings:
   a. feeding ethylene carbonate (EC) and methanol (ME) into the static mixer 1 in proportion and then into the preheater 2 to be preheated to the reaction temperature, wherein the molar ratio of ethylene carbonate to methanol ranges from 1:8 to 4:8; and then feeding the preheated reactants into the pre-reactor 3 together for pre-reaction;
   b. feeding the pre-treatment material exiting from the pre-reactor 3 into the reaction rectification column 4 for the catalytic rectification reaction, evaporating the azeotrope of dimethyl carbonate and methanol from the column top of the reaction rectification column 4, feeding ethylene glycol and excess methanol from the column bottom of the reaction rectification column 4 in the methanol recovery column 5; wherein the operating reflux ratio of the column top ranges from 1:1 to 3:1, and the filling volume percentage of the heterogeneous solid catalyst in the reaction rectification column ranges from 10% to 50%;
   c. the operating reflux ratio at the column top of methanol recovery column 5 ranges from 0:1 to 1:1;
   d. refining the crude ethylene glycol in the ethylene glycol rectification column 6, returning the light components (comprising ethylene glycol and methanol) at the column top to the reaction system, and extracting the ethylene glycol product from the side line of the column, wherein the ethylene glycol rectification column is operated under vacuum, and the residual pressure ranges from 0 to 5KPa.

Preferably, the reaction temperature in the preheater 2 and the pre-reactor 3 ranges from 70 to 100°C.

Preferably, the heterogeneous solid catalyst is a weak basic catalyst or a medium basic catalyst, and the shape thereof is spherical or columnar.

Preferably, the filling volume percentage of the heterogeneous solid catalyst in the reaction rectification column 4 ranges from 25% to 35%.

Preferably, a method for preparing the above weak basic catalyst comprises the following steps:
mixing 80 parts NaX molecular sieves and 20 parts NaY molecular sieve by weight uniformly, adding 10 parts calcium nitrate solution with 20% weight concentration and 50 parts sodium carbonate solution with 10% weight concentration therein and stirring evenly, drying the obtained product in an oven at 80°C for 10 hours and then drying the mixture in an oven at 150°C for 10 hours, then placing the dried product in a muffle furnace at 300°C for 8 hours' activation, and increasing to 500°C for 10 hours' activation, and after cooling down, placing the resulting product in a desiccator to cool for use.

Preferably, a method for preparing the above medium basic catalyst comprises the following steps:
slowly adding 20 parts potassium carbonate solution with 20% weight concentration and 60 parts cesium nitrate solution with 10% weight concentration in 100 parts γ-Al₂O₃ powder, and stirring evenly, then squeezing and cutting the obtained product into strips, drying the obtained product in an oven at 80°C for 10 hours and then drying the mixture in an oven at 150°C for 10 hours, then placing the dried product in a muffle furnace at 300°C for 8 hours' activation, and increasing to 550°C for 10 hours' activation, and after cooling down, placing the resulting product in a desiccator to cool for use.

### Example 1

Referring to Figure 1, the method for producing ethylene glycol using ethylene carbonate and methanol as raw materials mentioned in this Example comprises following preparation apparatus and preparation processes.
A. The preparation apparatus comprises followings: a static mixer 1, a preheater 2, a pre-reactor 3, a reaction rectification column 4, a methanol recovery column 5, and an ethylene glycol rectification column 6.

The static mixer 1 is connected with the pre-reactor 3 via the preheater 2, the upper part of pre-reactor 3 is connected with the upper portion of reaction rectification column 4, the bottom of reaction rectification column 4 is connected with the upper part of methanol recovery column 5 via a process pipeline, and the lower part of the methanol recovery column 5 is connected with the middle part of the ethylene glycol rectification column 6, the upper part of the ethylene glycol rectification column 6 is connected with the upper part of the methanol recovery column 5 to return light components therein; wherein the column bottom of the reaction rectification column 4 is also provided with a methanol-supplementing inlet, which is used to provide excess methanol during the catalytic rectification reaction, thereby achieving good reaction effect.

B. The preparation process comprises followings:
feeding ethylene carbonate (EC) and methanol (ME) into the static mixer 1 according to a molar ratio of 1:5 and then into the preheater 2 to be preheated to 65°C; and then feeding the preheated reactants into the pre-reactor 3 to contact with the solid catalyst 1# for performing reaction, which is then fed to the reaction rectification column 4 to contact with the solid catalyst 1# therein;
wherein the filling volume percentage of the solid catalyst 1# in the pre-reactor 3 is 80%, and the weight hourly space velocity of the reactant is 1.0h⁻¹ (i.e., the ratio of the mass flow rate of ethylene carbonate to the weight of solid catalyst is 1.0h⁻¹); and the filling volume percentage of the solid catalyst in the reaction rectification column 4 is 20%, and the dimethyl carbonate produced after reaction and the methanol supplemented from the column bottom form azeotropic components, which are extracted from the column top (the flow rate of supplemented methanol is 2000 kg/h) and the rectification temperature is 80°C. The operating reflux ratio is 1.3:1. The column bottom contains methanol and ethylene glycol (EG), and the typical composition in the column bottom is: ME in a range from 70% to 80%, and EG in a range from 20% to 30%;

The temperature at the column top of the methanol recovery column 5 is 65°C, the temperature of the column bottom thereof is 135°C, and the typical composition of the column bottom is: ME 5.2%, EG 94.8%;

The packing in the reaction rectification column 4 is CY700 gauze structured packing, with catalyst packing in the middle of the packing, and the number of plates in this Example is 55;
the packing in methanol recovery column 5 is CY700 structured packing, and the number of plates in this Example is 28; and
the packing in the ethylene glycol rectification column 6 is CY700 gauze structured packing, and the number of plates in this Example is 60.

In the ethylene glycol rectification column 6, the temperature of the column bottom is 125°C, the reflux ratio at the column top is 1.4, the pressure at the column top is 1.2KPa, and the pressure at the column bottom is 3.6 KPa;

The product extracted from the ethylene glycol rectification column 6 is cooled by a cooler and then extracted to a product intermediate tank. The EG content is ≥99.5%, typically is 99.78%. The extraction reflux ratio is 1.5:1;

In this Example, the solid catalyst 1# is weak basic solid catalyst with a spherical shape and an outer diameter of 3 mm. The feeding weight hourly space velocity of the raw materials (the mass flow rate of EC/the weight of the catalyst II in the reaction rectification column, that is, the space velocity of the pretreatment material) is 0.25h⁻¹.

The production process of the weak basic solid catalyst 1# is as follows: mixing 80 g NaX molecular sieve and 20 g NaY molecular sieve uniformly, adding 10 g calcium nitrate solution with 20% weight concentration and 50 g sodium carbonate solution with 10% weight concentration therein and stirring evenly, drying the obtained product in an oven at 80°C for 10 hours and then drying the mixture in an oven at 150°C for 10 hours, then placing the dried product in a muffle furnace at 300°C for 8 hours' activation, and increasing to 500°C for 10 hours' activation, and after cooling down, placing the resulting product in a desiccator to cool for use.

### Example 2

The method mentioned in this example uses ethylene carbonate and methanol as raw materials to produce ethylene glycol. The preparation apparatus is the same as that in Example 1. The preparation process is as follows:
feeding ethylene carbonate (EC) and methanol (ME) into the static mixer 1 according to a molar ratio of 1:6 and then into the preheater 2 to be preheated to 95°C; and then feeding the preheated reactants into the pre-reactor 3 to contact with the solid catalyst 2# for performing reaction, which is then fed to the reaction rectification column 4 to contact with the solid catalyst therein to react;
wherein the filling volume percentage of the solid catalyst 2# in the pre-reactor 3 is 85%, and the weight hourly space velocity of the reactant is 0.8 h⁻¹; and the filling percentage of the heterogeneous solid catalyst 2# in the reaction rectification column 4 is 30% and the weight hourly space velocity of the pre-treatment material is 0.15 h⁻¹, and the dimethyl carbonate produced after reaction and the methanol supplemented from the column bottom form azeotropic components, which are extracted from the column top and the rectification temperature is 65°C.

The operations of the methanol recovery column 5 and the ethylene glycol rectification column 6 are the same as those in Example 1.

In this Example, the solid catalyst 2# is a medium basic solid catalyst with a spherical shape and an outer diameter of 3 mm. The feeding weight hourly space velocity (calculated by EC) is 0.30 h⁻¹.

The production process of the solid catalyst 2# is as follows: slowly adding 20 g potassium carbonate solution with 20% weight concentration and 60 g cesium nitrate solution with 10% weight concentration in 100 g γ-Al₂O₃ powder, and stirring evenly, then squeezing and cutting the obtained product into strips, drying the obtained product in an oven at 80°C for 10 hours and then drying the mixture in an oven at 150°C for 10 hours, then placing the dried product in a muffle furnace at 300°C for 8 hours' activation, and increasing to 500°C for 10 hours' activation, and after cooling down, placing the resulting product in a desiccator to cool for use.

### Comparative Example 1

The preparation apparatus in this comparative example is partly identical to those in Example 1, and the identical contents will not be repeated.

The specific preparation process of ethylene glycol is as follows:
feeding respectively EC and methanol as the raw materials into an agitator-containing mixing tank with a solvent of 5000 L from the top thereof according to the molar ratio of 1:6, and feeding the sodium methoxide as catalyst (in the form of 30wt% sodium methoxide solution, the weight ratio of sodium methoxide to EC is 0.07) into the mixing tank through the other inlet of the mixing tank. The mixing tank has a stirring speed of 60 rpm/h. The lower part of the mixing tank is connected with a feeding pump which is used to feed the material to the reaction rectification column 4. The reaction rectification column is filled with CY gauze packing, and the supplemented methanol from the column bottom promotes EC conversion. The materials at the column bottom of the reaction rectification column enter the methanol recovery column 5, the methanol at the column top of the methanol recovery column 5 returns to the batching system, and the materials at the column bottom thereof enter the EG rectification column 6. The material at the column top of the EG rectification column returns to the feeding line of the methanol recovery column 5, the EG product is extracted from the column, and the material containing the sodium methoxide as catalyst is regularly extracted from the column bottom. Because the sodium methoxide can react with the carbon dioxide generated by the decomposition of ethylene carbonate to be converted into sodium carbonate, sodium methoxide and sodium carbonate and the like salts are separated from the ethylene glycol from the column bottom in the EG rectification system, and a small amount of higher polyalcohol is entrained. These materials become solid wastes of chemical plants. In this comparative example, 122 tons of solid wastes are generated every year. The solid waste needs to be delivered to a qualified unit for harmless treatment.

Taking the production apparatus with an annual output of 3500T ethylene glycol as an example, the effect comparison among Example 1, Example 2 and the conventional homogeneous catalyst in the prior art is shown in Table 1.

**Table 1 Effect Comparison Table**

| | EC conversion rate (%) | EG selectivity (%) | Catalyst consumption (t/a) | Amounts of solid wastes generated (t/a) | Catalyst consumption cost (ten thousand yuan/a) | Solid waste treatment cost (ten thousand yuan/a) |
|---|---|---|---|---|---|---|
| Example 1 | 99.8 | 97.2 | 2.4 | 2.4 | 24 | 1.2 |
| Example 2 | 99.9 | 97.5 | 2.4 | 2.4 | 24 | 1.2 |
| Compared Process | 99.0 | 96.2 | 122 | 122 | 122 | 61 |

In the present application, the 3500t/a EG production apparatus uses a total of 4.8 tons of solid catalyst. The solid catalyst has a lifespan of 2 years, that is, 2.4 tons of solid catalysts need to be replaced every year on average. These catalysts are also solid hazardous wastes that require qualified units to process. Compared with the homogeneous catalyst, the homogeneous catalyst produces 122 tons of solid wastes per year.

The advantages of the present application are shown in Table 1. The EC conversion rate is improved, the EG selectivity is also greatly improved, the catalyst consumption is greatly reduced, the amount of solid wastes is greatly reduced, the catalyst consumption cost is also greatly reduced, and the solid waste treatment cost is greatly reduced. In addition, compared with the homogeneous catalyst, the advantages of the solid catalyst used in the present application in terms of apparatus construction, material storage, material transportation and the like will not be repeated.

## Claims

1. A method for producing ethylene glycol using ethylene carbonate and methanol as raw materials comprising following steps:
a) performing pre-reaction of heated reactants to obtain a pre-treatment material, the reactants comprising ethylene carbonate and methanol;
b) subjecting the pre-treatment material to a catalytic rectification reaction to obtain a material stream I;
c) separating methanol from the material stream I to obtain a material stream II;
d) subjecting the material stream II to rectification to obtain a target product, the target product comprising ethylene glycol;
wherein, the catalytic rectification reaction is performed in the presence of a catalyst I;
the catalyst I comprising a solid catalyst.

2. The method according to claim 1**,** wherein, in step a), the pre-reaction is performed in the presence of catalyst II;
wherein, the catalyst II comprises a solid catalyst.

3. The method according to claim 2, wherein, in step a), a weight hourly space velocity of the reactants ranges from 0.8 to 1.0 h⁻¹;
preferably, a molar ratio of ethylene carbonate to methanol ranges from 1:4 to 1:8;
further preferably, a heating temperature in step a) ranges from 65 to 100°C.

4. The method according to claim 1, wherein, in step b), a weight hourly space velocity of the pre-treatment material ranges from 0.15 to 0.25 h⁻¹; and
preferably, the catalytic rectification reaction is performed at a temperature ranging from 65 to 80°C.

5. The method according to claim 1, wherein a shape of the solid catalyst is spherical or columnar.

6. The method according to claim 1, wherein the solid catalyst is basic catalyst.

7. The method according to claim 6, wherein, the basic catalyst is a molecular sieve catalyst;
the molecular sieve catalyst comprises element-modified molecular sieve,
wherein the element comprises at least one of an alkali metal element and an alkaline earth metal element, and
the molecular sieve comprises at least one of X-type molecular sieve, and Y-type molecular sieve;
preferably, the molecular sieve catalyst is prepared by following steps:
mixing a mixture comprising a molecular sieve with a solution containing a modification element source, which is then subjected to drying to obtain a precursor I; and
subjecting sequentially the precursor I to a first activation treatment and a second activation treatment to obtain the weak base catalyst;
wherein, the first activation treatment is performed at a temperature ranging from 250 to 350°C for a time ranging from 6 to 10 h; and
the second activation treatment is performed at a temperature ranging from 450 to 550°C for a time ranging from 8 to 12h;
further preferably, the molecular sieve is X-type molecular sieve and Y-type molecular sieve; and the modification element is alkali metal element and alkaline earth metal element.

8. The method according to claim 6, wherein the basic catalyst is a metal oxide catalyst;
the metal oxide catalyst comprises element-modified metal oxide;
wherein the element comprises at least one of alkali metals;
preferably, wherein the metal oxide catalyst is prepared by following steps:
mixing a material comprising a metal oxide with a solution comprising modification element source, which is subjected to drying to obtain a precursor II;
subjecting sequentially the precursor II to an activation treatment I and an activation treatment II to obtain a medium basic catalyst;
wherein, the activation treatment I is performed at a temperature ranging from 250 to 350°C for a time ranging from 6 to 10 h, and
the activation treatment II is performed at a temperature ranging from 500 to 600°C for a time ranging from 8 to 12 h;
further preferably, wherein the metal oxide is γ-Al₂O₃.

9. The method according to claim 1, wherein, in step d), the material stream II is rectified under vacuum environment; and
the vacuum environment ranges from 0 to 5 KPa.

10. The method according to claim 1, wherein a content of the ethylene glycol in the target product is ≥99.5%.

11. The method according to claim 1 comprising following steps:
a) feeding the reactants heated by a preheater into a pre-reactor for pre-reaction to obtain the pre-treatment material, the reactants comprising ethylene carbonate and methanol;
b) feeding the pre-treatment material into a reaction rectification column for a catalytic rectification reaction to obtain the material stream I;
c) feeding the material stream I into a methanol recovery column to separate methanol in the material stream I to obtain the material stream II;
d) feeding the material stream II into an ethylene glycol rectification column for rectification to obtain a target product, the target product comprising ethylene glycol;
wherein, the catalytic rectification reaction is performed in the presence of the catalyst I;
the catalyst I comprising the solid catalyst.

12. An apparatus for producing ethylene glycol using ethylene carbonate and methanol as raw materials according to any method of claims 1 to 11 comprising a raw material mixing unit, a preheating unit, a pre-reaction unit, a reaction rectification unit, a methanol recovery unit and an ethylene glycol rectification unit;
the raw material mixing unit, the preheating unit and the pre-reaction unit are connected in sequence;
an outlet of the pre-reaction unit is connected with an upper end of the reaction rectification unit;
a lower end of the reaction rectification unit is connected with an upper end of the methanol recovery unit;
a lower end of the methanol recovery unit is connected with the ethylene glycol rectification unit; and
the ethylene glycol rectification unit is provided with a target product outlet.

13. The apparatus according to claim 12, wherein the apparatus comprises a raw material mixer, a preheater, a pre-reactor, a reaction rectification column, a methanol recovery column, and an ethylene glycol rectification column;
the raw material mixer, the preheater and the pre-reactor are connected in sequence;
an outlet of the pre-reactor is connected with an upper end of the reaction rectification column;
a lower end of the reaction rectification column is connected with an upper end of the methanol recovery column;
a lower end of the methanol recovery column is connected with the ethylene glycol rectification column; and
a middle part of the ethylene glycol rectification column is provided with a target product outlet;
preferably, the lower end of the reaction rectification column is also provided with a methanol-supplementing inlet;
further preferably, an upper end of the ethylene glycol rectification column is connected with the upper end of the methanol recovery column.

14. The apparatus according to claim 13, wherein, the pre-reactor is filled with catalyst II; and
a filling volume percentage of the catalyst II in the pre-reactor ranges from 70% to 90%.

15. The apparatus according to claim 13, wherein the reaction rectification column is filled with catalyst I, and
a filling volume percentage of the catalyst I in the reaction rectification column ranges from 10% to 50%.

## Patentansprüche

1. Ein Verfahren zur Herstellung von Ethylenglykol unter Verwendung von Ethylencarbonat und Methanol als Rohstoffe, umfassend folgende Schritte:
a) Durchführung einer Vorreaktion der erhitzten Edukte zur Gewinnung eines Vorbehandlungsmaterials, wobei die Edukte Ethylencarbonat und Methanol umfassen;
b) Unterziehen des Vorbehandlungsmaterials einer katalytischen Rektifikationsreaktion zur Gewinnung eines Materialstroms I;
c) Abtrennen von Methanol aus dem Materialstrom I zur Gewinnung eines Materialstroms II;
d) Unterziehen des Materialstroms II einer Rektifikation zur Gewinnung eines Zielprodukts, wobei das Zielprodukt Ethylenglykol umfasst;
wobei die katalytische Rektifikationsreaktion in Anwesenheit eines Katalysators I durchgeführt wird;
-- der Katalysator I einen festen Katalysator umfasst.

2. Das Verfahren nach Anspruch 1, wobei in Schritt a) die Vorreaktion in Anwesenheit eines Katalysators II durchgeführt wird;
- wobei der Katalysator II einen festen Katalysator umfasst.

3. Das Verfahren nach Anspruch 2, wobei in Schritt a) eine Gewichtsstundenraumgeschwindigkeit der Edukte im Bereich von 0,8 bis 1,0 h⁻¹ liegt;
- vorzugsweise liegt das molare Verhältnis von Ethylencarbonat zu Methanol im Bereich von 1:4 bis 1:8;
- weiter vorzugsweise liegt die Heiztemperatur in Schritt a) im Bereich von 65 bis 100 °C.

4. Das Verfahren nach Anspruch 1, wobei in Schritt b) eine Gewichtsstundenraumgeschwindigkeit des Vorbehandlungsmaterials im Bereich von 0,15 bis 0,25 h⁻¹ liegt;
- vorzugsweise wird die katalytische Rektifikationsreaktion bei einer Temperatur im Bereich von 65 bis 80 °C durchgeführt.

5. Das Verfahren nach Anspruch 1, wobei die Form des festen Katalysators kugelförmig oder zylinderförmig ist.

6. Das Verfahren nach Anspruch 1, wobei der feste Katalysator ein basischer Katalysator ist.

7. Das Verfahren nach Anspruch 6, wobei
- der basische Katalysator ein Molekularsieb-Katalysator ist;
- der Molekularsieb-Katalysator ein elementmodifiziertes Molekularsieb umfasst, wobei das Element mindestens eines der Elemente aus der Gruppe der Alkalimetalle und Erdalkalimetalle umfasst, und
- das Molekularsieb mindestens eines der folgenden umfasst: X-Typ Molekularsieb und Y-Typ Molekularsieb;
- vorzugsweise wird der Molekularsieb-Katalysator wie folgt hergestellt:
-- Mischen eines Gemischs, das ein Molekularsieb umfasst, mit einer Lösung, die eine Quelle des Modifikationselements enthält, gefolgt von Trocknung zur Gewinnung eines Vorprodukts I; und
-- sequentielles Unterziehen des Vorprodukts I einer ersten Aktivierungsbehandlung und einer zweiten Aktivierungsbehandlung zur Herstellung des schwach basischen Katalysators;
- wobei die erste Aktivierungsbehandlung bei einer Temperatur von 250 bis 350 °C für eine Dauer von 6 bis 10 Stunden erfolgt; und die zweite Aktivierungsbehandlung bei 450 bis 550 °C für 8 bis 12 Stunden erfolgt;
- weiter vorzugsweise ist das Molekularsieb ein X-Typ und Y-Typ Molekularsieb; und das Modifikationselement ein Alkalimetall- und Erdalkalimetallelement.

8. Das Verfahren nach Anspruch 6, wobei der basische Katalysator ein Metalloxid-Katalysator ist;
- der Metalloxid-Katalysator ein elementmodifiziertes Metalloxid umfasst;
- wobei das Element mindestens eines der Alkalimetalle umfasst;
- vorzugsweise wird der Metalloxid-Katalysator wie folgt hergestellt:
-- Mischen eines Materials, das ein Metalloxid umfasst, mit einer Lösung, die eine Modifikationselementquelle umfasst, gefolgt von Trocknung zur Gewinnung eines Vorprodukts II;
-- sequentielles Unterziehen des Vorprodukts II einer Aktivierungsbehandlung I und einer Aktivierungsbehandlung II zur Herstellung eines mittelbasischen Katalysators;
- wobei die Aktivierungsbehandlung I bei 250 bis 350 °C für 6 bis 10 Stunden erfolgt, und
- die Aktivierungsbehandlung II bei 500 bis 600 °C für 8 bis 12 Stunden erfolgt;
-- weiter vorzugsweise ist das Metalloxid γ-Al₂O₃.

9. Das Verfahren nach Anspruch 1, wobei in Schritt d) der Materialstrom II unter Vakuumbedingungen rektifiziert wird;
- die Vakuumbedingungen liegen im Bereich von 0 bis 5 kPa.

10. Das Verfahren nach Anspruch 1, wobei der Gehalt an Ethylenglykol im Zielprodukt ≥ 99,5 % beträgt.

11. Das Verfahren nach Anspruch 1, umfassend folgende Schritte:
a) Zuführen der durch einen Vorwärmer erhitzten Edukte in einen Vorreaktor zur Vorreaktion zur Gewinnung des Vorbehandlungsmaterials, wobei die Edukte Ethylencarbonat und Methanol umfassen;
b) Zuführen des Vorbehandlungsmaterials in eine Reaktionsrektifikationskolonne zur katalytischen Rektifikationsreaktion zur Gewinnung des Materialstroms I;
c) Zuführen des Materialstroms I in eine Methanolrückgewinnungskolonne zur Trennung von Methanol im Materialstrom I zur Gewinnung des Materialstroms II;
d) Zuführen des Materialstroms II in eine Ethylenglykolrektifikationskolonne zur Rektifikation zur Gewinnung des Zielprodukts, wobei das Zielprodukt Ethylenglykol umfasst;
- wobei die katalytische Rektifikationsreaktion in Anwesenheit des Katalysators I durchgeführt wird;
-- der Katalysator I umfasst den festen Katalysator.

12. Eine Vorrichtung zur Herstellung von Ethylenglykol unter Verwendung von Ethylencarbonat und Methanol als Rohstoffe gemäß einem Verfahren nach einem der Ansprüche 1 bis 11, umfassend eine Rohstoffmischeinheit, eine Vorheizeinheit, eine Vorreaktionseinheit, eine Reaktionsrektifikationseinheit, eine Methanolrückgewinnungseinheit und eine Ethylenglykolrektifikationseinheit;
- die Rohstoffmischeinheit, die Vorheizeinheit und die Vorreaktionseinheit sind in Serie verbunden;
- ein Ausgang der Vorreaktionseinheit ist mit dem oberen Ende der Reaktionsrektifikationseinheit verbunden;
- ein unteres Ende der Reaktionsrektifikationseinheit ist mit dem oberen Ende der Methanolrückgewinnungseinheit verbunden;
- ein unteres Ende der Methanolrückgewinnungseinheit ist mit der Ethylenglykolrektifikationseinheit verbunden;
- die Ethylenglykolrektifikationseinheit ist mit einem Zielproduktauslass versehen.

13. Die Vorrichtung nach Anspruch 12, wobei die Vorrichtung einen Rohstoffmischer, einen Vorwärmer, einen Vorreaktor, eine Reaktionsrektifikationskolonne, eine Methanolrückgewinnungskolonne und eine Ethylenglykolrektifikationskolonne umfasst;
- der Rohstoffmischer, der Vorwärmer und der Vorreaktor sind in Serie verbunden;
- ein Ausgang des Vorreaktors ist mit dem oberen Ende der Reaktionsrektifikationskolonne verbunden;
- ein unteres Ende der Reaktionsrektifikationskolonne ist mit dem oberen Ende der Methanolrückgewinnungskolonne verbunden;
- ein unteres Ende der Methanolrückgewinnungskolonne ist mit der Ethylenglykolrektifikationskolonne verbunden;
- ein mittlerer Teil der Ethylenglykolrektifikationskolonne ist mit einem Zielproduktauslass versehen;
- vorzugsweise ist das untere Ende der Reaktionsrektifikationskolonne auch mit einem Methanolzuführeinlass versehen;
- weiter vorzugsweise ist ein oberes Ende der Ethylenglykolrektifikationskolonne mit dem oberen Ende der Methanolrückgewinnungskolonne verbunden.

14. Die Vorrichtung nach Anspruch 13, wobei der Vorreaktor mit Katalysator II gefüllt ist;
- der Füllvolumenprozentsatz des Katalysators II im Vorreaktor liegt im Bereich von 70 % bis 90 %.

15. Die Vorrichtung nach Anspruch 13, wobei die Reaktionsrektifikationskolonne mit Katalysator I gefüllt ist;
- der Füllvolumenprozentsatz des Katalysators I in der Reaktionsrektifikationskolonne liegt im Bereich von 10 % bis 50 %.

## Revendications

1. Un procédé de production de glycol d'éthylène utilisant le carbonate d'éthylène et le méthanol comme matières premières, comprenant les étapes suivantes :
a) effectuer une pré-réaction des réactifs chauffés afin d'obtenir un matériau de prétraitement, les réactifs comprenant du carbonate d'éthylène et du méthanol ;
b) soumettre le matériau de prétraitement à une réaction de rectification catalytique afin d'obtenir un flux de matière I ;
c) séparer le méthanol du flux de matière I afin d'obtenir un flux de matière II ;
d) soumettre le flux de matière II à une rectification afin d'obtenir un produit cible, ledit produit cible comprenant du glycol d'éthylène ;
la réaction de rectification catalytique étant réalisée en présence d'un catalyseur I ;
-- le catalyseur I comprenant un catalyseur solide.

2. Le procédé selon la revendication 1, dans lequel, à l'étape a), la pré-réaction est réalisée en présence d'un catalyseur II ;
- le catalyseur II comprenant un catalyseur solide.

3. Le procédé selon la revendication 2, dans lequel, à l'étape a), la vitesse spatiale horaire pondérale des réactifs est comprise entre 0,8 et 1,0 h⁻¹ ;
- de préférence, le rapport molaire de carbonate d'éthylène à méthanol est compris entre 1:4 et 1:8 ;
- plus préférentiellement, la température de chauffage à l'étape a) est comprise entre 65 et 100 °C.

4. Le procédé selon la revendication 1, dans lequel, à l'étape b), la vitesse spatiale horaire pondérale du matériau de prétraitement est comprise entre 0,15 et 0,25 h⁻¹ ;
- de préférence, la réaction de rectification catalytique est réalisée à une température comprise entre 65 et 80 °C.

5. Le procédé selon la revendication 1, dans lequel la forme du catalyseur solide est sphérique ou en forme de colonne.

6. Le procédé selon la revendication 1, dans lequel le catalyseur solide est un catalyseur basique.

7. Le procédé selon la revendication 6, dans lequel le catalyseur basique est un catalyseur à tamis moléculaire ;
- le catalyseur à tamis moléculaire comprenant un tamis moléculaire modifié par un élément, lequel comprend au moins un élément alcalin ou alcalino-terreux ;
- le tamis moléculaire comprenant au moins un tamis de type X ou de type Y ;
- de préférence, le catalyseur à tamis moléculaire est préparé selon les étapes suivantes :
-- mélange d'un mélange contenant un tamis moléculaire avec une solution contenant une source d'élément de modification, suivi d'un séchage pour obtenir un précurseur I ;
-- soumission du précurseur I à un premier traitement d'activation puis à un second traitement d'activation pour obtenir un catalyseur faiblement basique ;
- le premier traitement d'activation étant effectué à une température comprise entre 250 et 350 °C pendant 6 à 10 heures ; et le second traitement entre 450 et 550 °C pendant 8 à 12 heures ;
- plus préférentiellement, le tamis moléculaire est un tamis de type X et de type Y, et l'élément de modification est un métal alcalin ou alcalino-terreux.

8. Le procédé selon la revendication 6, dans lequel le catalyseur basique est un catalyseur à oxyde métallique ;
- le catalyseur à oxyde métallique comprenant un oxyde métallique modifié par un élément ;
- l'élément comprenant au moins un métal alcalin ;
- de préférence, le catalyseur à oxyde métallique est préparé selon les étapes suivantes :
-- mélange d'un matériau contenant un oxyde métallique avec une solution contenant une source d'élément de modification, suivi d'un séchage pour obtenir un précurseur II ;
-- soumission séquentielle du précurseur II à un traitement d'activation I puis à un traitement d'activation II pour obtenir un catalyseur modérément basique ;
- le traitement I est effectué à une température de 250 à 350 °C pendant 6 à 10 heures ;
- le traitement II est effectué à une température de 500 à 600 °C pendant 8 à 12 heures ;
-- plus préférentiellement, l'oxyde métallique est le γ-Al₂O₃.

9. Le procédé selon la revendication 1, dans lequel, à l'étape d), le flux de matière II est rectifié sous vide ;
- l'environnement sous vide étant compris entre 0 et 5 kPa.

10. Le procédé selon la revendication 1, dans lequel la teneur en glycol d'éthylène dans le produit cible est supérieure ou égale à 99,5 %.

11. Le procédé selon la revendication 1, comprenant les étapes suivantes :
a) introduction des réactifs chauffés par un préchauffeur dans un pré-réacteur pour réaliser une pré-réaction et obtenir le matériau de prétraitement, les réactifs comprenant du carbonate d'éthylène et du méthanol ;
b) introduction du matériau de prétraitement dans une colonne de rectification réactionnelle pour effectuer une réaction de rectification catalytique afin d'obtenir le flux de matière I ;
c) introduction du flux de matière I dans une colonne de récupération du méthanol pour séparer le méthanol et obtenir le flux de matière II ;
d) introduction du flux de matière II dans une colonne de rectification du glycol d'éthylène pour obtenir le produit cible, lequel contient du glycol d'éthylène ;
- la réaction de rectification catalytique étant réalisée en présence du catalyseur I ;
-- le catalyseur I comprenant un catalyseur solide.

12. Un appareil pour la production de glycol d'éthylène à partir de carbonate d'éthylène et de méthanol selon l'un quelconque des procédés des revendications 1 à 11, comprenant une unité de mélange des matières premières, une unité de préchauffage, une unité de pré-réaction, une unité de rectification réactionnelle, une unité de récupération du méthanol et une unité de rectification du glycol d'éthylène ;
- l'unité de mélange, l'unité de préchauffage et l'unité de pré-réaction étant connectées en série ;
- la sortie de l'unité de pré-réaction est connectée à l'extrémité supérieure de l'unité de rectification réactionnelle ;
- l'extrémité inférieure de l'unité de rectification réactionnelle est connectée à l'extrémité supérieure de l'unité de récupération du méthanol ;
- l'extrémité inférieure de l'unité de récupération du méthanol est connectée à l'unité de rectification du glycol d'éthylène ;
- l'unité de rectification du glycol d'éthylène est munie d'une sortie pour le produit cible.

13. L'appareil selon la revendication 12, dans lequel l'appareil comprend un mélangeur de matières premières, un préchauffeur, un pré-réacteur, une colonne de rectification réactionnelle, une colonne de récupération du méthanol et une colonne de rectification du glycol d'éthylène ;
- le mélangeur, le préchauffeur et le pré-réacteur sont connectés en série ;
- la sortie du pré-réacteur est connectée à l'extrémité supérieure de la colonne de rectification réactionnelle ;
- l'extrémité inférieure de la colonne de rectification réactionnelle est connectée à l'extrémité supérieure de la colonne de récupération du méthanol ;
- l'extrémité inférieure de la colonne de récupération du méthanol est connectée à la colonne de rectification du glycol d'éthylène ;
- une partie intermédiaire de la colonne de rectification du glycol d'éthylène est munie d'une sortie pour le produit cible ;
- de préférence, l'extrémité inférieure de la colonne de rectification réactionnelle est également pourvue d'une entrée pour l'appoint en méthanol ;
- plus préférentiellement, l'extrémité supérieure de la colonne de rectification du glycol d'éthylène est connectée à l'extrémité supérieure de la colonne de récupération du méthanol.

14. L'appareil selon la revendication 13, dans lequel le pré-réacteur est rempli de catalyseur II ;
- le pourcentage de remplissage volumique du catalyseur II dans le pré-réacteur est compris entre 70 % et 90 %.

15. L'appareil selon la revendication 13, dans lequel la colonne de rectification réactionnelle est remplie de catalyseur I ;
- le pourcentage de remplissage volumique du catalyseur I dans la colonne de rectification réactionnelle est compris entre 10 % et 50 %.
